# EUROPEAN PATENT SPECIFICATION

(11) **EP 1 325 060 B1**
(45) Date of publication and mention of the grant of the patent: **09.04.2008**
(21) Application number: 01956655.3
(22) Date of filing: 01.08.2001
(51) Int. Cl.: C08H 1/00, A61L 15/40, A61L 31/00, A61L 27/36, A61K 47/46, A61L 31/04

(54) **SOYBEAN-BASED THERMOPLASTICS AS BIOMATERIALS**
THERMOPLASTE AUF DER BASIS VON SOJABOHNEN ALS BIOMATERIALIEN
THERMOPLASTIQUES A BASE DE SOJA UTILISES COMME BIOMATERIAUX

(30) Priority: 02.08.2000 GB 0018953
(43) Date of publication of application: 09.07.2003
(73) Proprietor: University of Brighton, Brighton, East Sussex BN2 4AT (GB)
(72) Inventor: SANTIN, Matteo, School of Pharmacy & Biomolecular Sciences, Brighton, Sussex BN2 4GJ (GB); NICOLAIS, Luigi, Centro di Ricerca Interdisciplinare sui Biom., 80125 Naples (IT); AMBROSIO, Luigi, Centro di Ricerca Interdisciplinare sui Biom., 80125 Naples (IT)
(74) Representative: Gaunt, Robert John
(86) International application number: PCT/GB2001/003464
(87) International publication number: WO 2002/010261

(56) References cited:
- US-A- 4 511 588
- US-A- 4 897 280
- US-A- 5 935 996
- DATABASE CHEMABS [Online] CHEMICAL ABSTRACTS SERVICE, COLUMBUE, OHIO, US; "Method for production of molded product using food waste and biodegradable polymer." retrieved from STN Database accession no. 132-352157 CA XP002180276 & JP 00 141396 A (BIPP K.K.) 23 May 2000 (2000-05-23)
- DATABASE CHEMABS [Online] Chemical Abstracts Service, Columbus, Ohio, US; "Manufacture of composite resin foams using moist plant residue" retrieved from STN Database accession no. 127:279256 CA XP002180277 & JP 09 249761 A (DAIKO K.K.) 22 September 1997 (1997-09-22)
- HWANG K. ET AL.: "The effect of topical genistein on soft tissue wound healing in rats." JOURNAL OF HISTOTECHNOLOGY, vol. 24, no. 2, 2001, pages 95-99, XP001032945 usa

## Description

This invention relates to soybean-based thermoset biomaterials. They are produced by a method which comprises the thermosetting of soybean de-fatted tofu. The biomaterials are useful in various biomedical applications, including bone fillers, wound dressings and temporary barriers to prevent post-surgical tissue adherence.

### BACKGROUND

The production of new biodegradable materials, both of synthetic and natural sources, is an important goal in biomedical applications where the implant has to perform a temporary function in the body. Tissue in-growth and drug delivery are usually associated with the use of completely degradable biomaterials unless a protracted scaffolding action is required'. However, the ideal biodegradable material has not been achieved yet due to two major drawbacks: (a) the difficult modulation of the degradation time; (b) the possible toxic effects of the degradation products.

The degradation of the materials is generally based on three main events: (1) the spontaneous hydrolysis of the chemical bonds supporting the polymeric structure; (2) the mechanical action exerted by the in-growth of the surrounding tissues; (3) the inflammatory response elicited by the foreign material.

The modulation of the degradation is often very difficult to obtain since it can be affected by the individual variability of patients. For example, a young subject's tissue growth is faster than elderly tissue regeneration and patients with particular diseases can produce altered inflammatory responses. The toxicity problems often related to the degradation products of synthetic materials have been partially bypassed by the use of natural polymers². These materials, however, bring other kinds of disadvantages such as the purification costs and the risks of transmittable diseases or allergies related to their use.

The ideal biodegradable polymer should be a polymer degrading into natural and compatible molecules through spontaneous hydrolysis. In addition, the material exposed surface should support the adhesion of tissue cells to facilitate tissue in-growth. A reduced immune response should be coupled to both material implantation and its degradation process.

Poly(lactic acid), poly(glycolic acid) and poly(lactide-co-glycolide) are a special class of aliphatic polyester which can be synthesised from lactic and glycolic acid³. The advantage of using this class of biomaterials is that, although synthetic, they degrade into molecules which are normally produced by the body metabolism and, therefore, are not harmful. Furthermore, these materials are easily processed into films and threads as well as into micro- and nano-particles. The physical-chemical degradation of the poly(lactide-co-glycolide) co-polymers can also be modulated through the percentage of the two monomers and the molecular weight of the final polymer.

This class of polymers can be considered as the best biodegradable materials in terms of biocompatibility, although they are expensive and elicit an inflammatory response which can be more or less pronounced depending on their molecular weight. Furthermore, these materials do not support cell growth unless they are appropriately functionalised and some studies suggest a certain degree of cytotoxicity when poly(lactic acid) is added to cell cultures at relatively high concentrations³.

Nevertheless, poly(lactic acid)- and poly(glycolic acid)-based products are considered as the best biodegradable biomaterials on the market for several biomedical applications. For example, biodegradable suturing materials and biodegradable barriers in dental practise are obtained from these polymers.

Soybean proteins have been recently included among the natural and biodegradable polymers which can be processed into films for engineering applications. A patent has claimed the production of thermoplastics from soybean proteins⁴ and only recently their use as biomaterials has been suggested⁵.

When deprived of its oil component, soybean flour is a natural composite mainly constituted by proteins and carbohydrates. The production of the soybean milk from the ground flour and its processing into cheese of different texture by calcium solutions have been explored in the food industry to provide healthy alimentary products⁶. To our knowledge no report has been published about the production of thermoplastics and thermoset materials from soybean cheese (tofu) and their use in the biomedical field. Relying on the know-how of the food industry, methods for the production and the sterilisation of tofu-based thermoplastics and thermoset materials can be easily applied to the preparation of novel biomaterials^{6,7}.

### THE INVENTION

This invention is based on the discovery that films of a soybean-type material can induce an inhibition of the inflammatory response and they therefore degrade in the physiological environment principally on the basis of their hydrolysis. The thermoplastics and thermoset materials, made of de-fatted extra-firm tofu, are also able to induce a fast formation (2 days) of a mineral phase with a chemical composition similar to the bone hydroxylapatite when incubated in physiological buffer with a salt composition similar to that of the bone exudates. This mineralization process precedes the film biodegradation which begins only after 3 days of incubation and proceeds with the formation of a porosity evenly distributed throughout the exposed surface. Although the inventors have demonstrated these effects only *in vitro,* the congruency of the data obtained from different experimental procedures allows one to predict a similar behaviour *in vivo.* The possibility to modify both the chemistry and the morphology of the materials are also claimed in the present invention offering a series of approaches to make them suitable for many biomedical applications. These modifications aim to modulate the degradation time of the material by varying its porosity and surface chemistry, as well as to improve the material's biocompatibility and mechanical properties. The inhibition of the inflammatory cell activation by the thermoplastics, the thermoset materials and their degradation products indicates that the degradation of the material *in vivo* will be affected only by its spontaneous hydrolysis and by the tissue in-growth both guided by the exposed surface of the implant. Thermoplastics made of tofu also favoured cell adhesion and proliferation. Thermoplastics made of tofu can be, therefore, used both as monolith and as coating material to encourage the formation of new tissues and the inhibition of the implant-related inflammation.

The tofu-based or derived thermoset products of the present invention are biomaterials. As understood by persons skilled in the art, a biomaterial is a non-viable material used in a medical device intended to interact with biological systems.

The thermoset biomaterials of the present invention can be produced by a method which comprises the thermosetting of soybean de-fatted tofu. In one embodiment of the method, the soybean curd (tofu) is prepared from defatted soybean milk by a coagulation process (e.g. a calcium-induced coagulation process). The de-fatted tofu may be moulded or sliced to the desired shape and size and then incubated at an elevated temperature, or alternatively autoclaved at an elevated temperature, until thermosetting is complete.

Main areas of applications of these materials will be wound healing, skeletal and dental osteointegration, post-surgical tissue adherence and drug delivery. More specifically, the thermoset biomaterials may be used as components of wound dressings, temporary barriers to prevent post-surgical tissue adherence, fillers or coating materials with osteointegrative potential (i.e. which can become integrated into bone) and anti-inflammatory agents.

The present invention will now be further illustrated by the following Examples and with reference to the accompanying figures, in which:-
Figure 1 shows scanning electron microscopy of compact tofu thermoplastic in a dry state (a) fracture surface (magnification x12,000); (b) exposed surface (magnification x3,000).
Figure 2 shows scanning electron microscopy of tofu thermoplastics swollen in PBS and autoclaved at different pressure values. (a) 1 Psi (0.1 Bar) fracture surface; (b) 9 Psi (0.6 Bar) fracture surface; (c) 14 Psi (1 Bar) fracture surface; (d) 1 Psi (0.1 Bar) exposed surface; (e) 9 Psi (0.6 Bar) exposed surface; (1) 14 Psi (1 Bar) exposed surface. Magnification x12,000.
Figure 3 shows scanning electron microscopy of PBS-swollen and freeze-dried tofu thermoplastic. Magnification x12,000.
Figure 4 shows water uptake of tofu thermoplastic films after incubation in physiological solution. Experiments were carried out in triplicate.
Figure 5 shows a profile of tofu thermoplastic film degradation in physiological buffer. Experiments were carried out in triplicate.
Figure 6 shows scanning electron microscopy of degrading tofu thermoplastics after 7 days in physiological buffer. (a) degrading polymer skin; (b) underlying material bulk. Magnification x200.
Figure 7 shows cryo-scanning electron microscopy of tofu thermoplastic film surface. (a) control film; (b) 2-days incubation in simulating body fluid at 37°C. Magnification x3,000.
Figure 8 shows elemental dispersive x-ray analysis of tofu thermoplastic films. (a) control; (b) 2-days incubation in simulated body fluid at 37°C.
Figure 9 shows adsorbed levels of proteins involved in the wound healing process. (a) C3 fragment of complement system; (b) fibrinogen; (c) immunoglobulins; (d) fibronectin. Data are expressed as mean ± standard deviation from n=6. Asterisk indicates material significantly different from tofu at p<0.05; double asterisk indicates material significantly different from tofu at p<0.01.
Figure 10 shows the effect of tofu thermoplastics and their extracts on the free radical production by mononuclear cells. Control is the spontaneous activation of the cells obtained during the experiment. Data are expressed as mean ± standard deviation from n=6. Samples were significantly different from the control cells at p<0.01.
Figure 11 shows adhesion of monocytes/macrophages on tofu films analysed by scanning electron microscopy. (a) isolated round-shaped cells; (b) cell cluster; (c) isolated spread cells. Bars = 3µm.
Figure 12 shows adhesion and proliferation of 3T3 murine fibroblasts on soybean-based thermoplastics. (a) control 6 h; (b) tofu 6 h; (c) control 24 h; (d) tofu 6 h; (e) control 48 h; (f) tofu 48 h. Experiments were performed in triplicate.
Figure 13 shows 3T3 murine fibroblast spreading on tofu-based thermoplastic after 24-h incubation time.

### EXAMPLE 1: Preparation of compact specimens at high temperature.

### METHOD

Slices of de-fatted soybean extra-firm cheese, which is prepared by conventional coagulation methods with CaCl₂ solutions (10-40 mM)⁸ or obtained from commercial tofu, are cut by sharp blade at a uniform thickness (1.0 cm).

The films obtained are incubated in an oven at 90 °C until complete thermosetting.

In an alternative method, the thermosetting was obtained by microwave baking.

The method is also applicable to the preparation of specimens with different morphologies by preparation of the soybean cheese in appropriate moulds. Cubes, disks, membranes or extruded fibres can be thus obtained. Microparticles can also be prepared by crumbling either fresh tofu or its thermoplastics in a blender. In the first case the crumbled material undergoes thermosetting as reported in this Example and in Example 2.

The specimens, prepared according to this method, were sputter-coated with gold and analysed by Scanning Electron Microscopy at x3,000 and x12,000, 5 kV.

### RESULTS

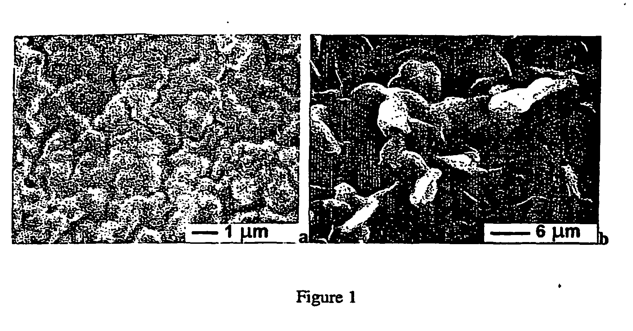

Rigid films of 1.0 ± 0.2 mm thickness were obtained. The morphology of the film fracture surface appeared to be very compact with a roughness visible only at high magnification (Figure 1 a). The exposed surface showed a roughness in which peaks and troughs were already visible at x3000 magnification (Figure 1 b).

### EXAMPLE 2: Preparation of expanded tofu gels at different pressures.

### METHOD

Tofu slices were immersed in phosphate buffered saline pH 7.2 (PBS) for 24 h at room temperature and autoclaved at different temperatures (100, 115; 121 °C). Each temperature value corresponded to a different pressure (100°C = 1 Psi (0.1 Bar); 115 °C = 9 Psi (0.6 Bar); 121 °C = 14 Psi (1 Bar)). The gel slices were kept for 5 min at the settling temperature and the pressure of the autoclave was then gradually decreased. The films were finally freeze-dried overnight.

The same procedure was applied to the fresh tofu cheese specimens⁸.

In an alternative method, the wet films were freeze-dried overnight.

All the specimens were sputter-coated with gold and analysed by Scanning Electron Microscopy at x12,000, 5 kV.

### RESULTS

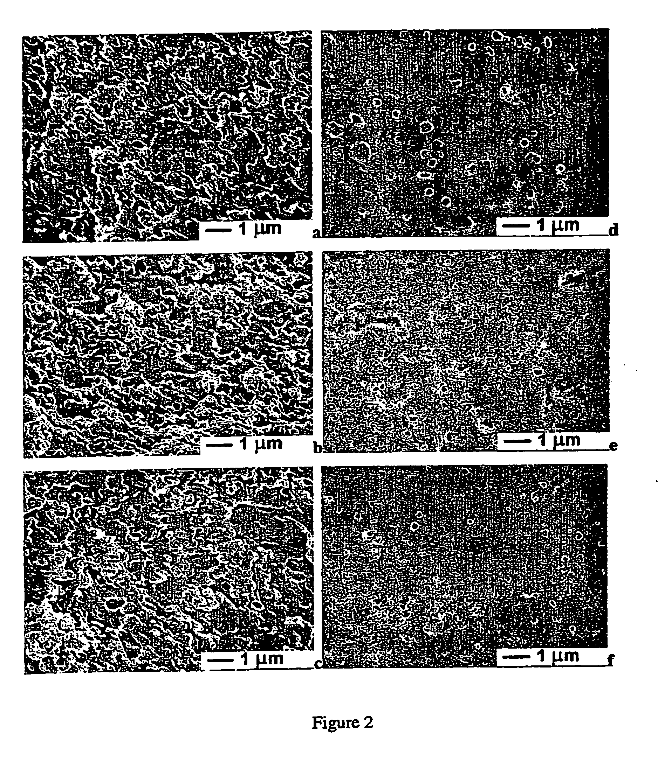

By the adopted autoclaving procedures gels of different porosity were obtained. The pore density increased with the increase of the autoclaving temperature and pressure on both the fracture and exposed surfaces, but the exposed surfaces were less porous than the fracture ones (Figure 2 a-f). No difference was found in terms of pore diameter (ca. 200 nm) at the different autoclaving conditions.

The exposed surface of the PBS-swollen and autoclaved materials lost the rough morphology observed in the dry control (Figure 1 b) perhaps as a consequence of the swelling process.

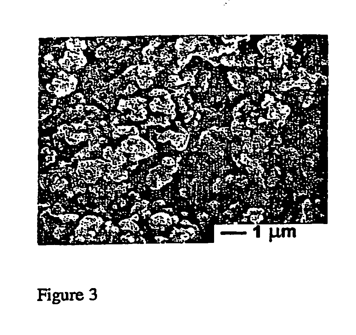

The simple freeze-drying of the films produced a different morphology where no pore was visible, but the roughness of the exposed surface was enhanced by the formation of protruding beads (Figure 3).

Porous specimens of different sizes and morphologies can be obtained as reported in Example 1 (i.e. cubes, membranes, particles, rods, disks).

### EXAMPLE 3: Preparation of polypeptide-functionalised tofu thermoplastics.

### METHOD

Tofu-derived thermoplastics were functionalised by grafting specific bioactive peptides to the soybean polymers through the reaction of aldehydes with amino groups of the soybean proteins (Schiff's bases formation). The films were immersed in a 0.25% (w/v) glutaraldehyde solution in phosphate buffer containing different concentrations of the bioactive peptides for 1 h at room temperature. Alternatively, 0.38% (w/v) formaldehyde was used as coupling reagent. In both the protocols, a step of reduction of the double C=N bonds by 10 mM CNBr was performed.

Alternatively, grafting can be achieved via disulphide bridges or via alcohol esterification or through other classical biochemical methods

All the peptides with specific cell receptor functions, calcium-binding properties as well as growth factors are included in this invention as functionalisation molecules of the tofu-derived thermoplastics.

### EXAMPLE 4: Tofu thermoplastic/thermoset composite materials.

### METHOD

The synthesis of composite biomaterials based on tofu thermoplastics is based on techniques such as blending, interpenetrating polymer networks and grafting with biocompatible synthetic and natural polymers which can confer to the tofu thermoplastics:
(i) Increased plasticity (low glass transition temperature) at a dry state;
(ii) Increased hydrophobicity;
(iii) Increased hydrophilicity (increased swelling properties in an aqueous environment);
(iv) Physical and chemical cross-linking;
(v) Physical and biological (osteointegration) properties
   (i) and (ii) Tofu cheese extra-firm paste is blended at 90 °C with 1% (w/v) melted polycaprolactone of different molecular weights.
   (iii) Tofu-derived porous thermoplastics, prepared as reported in Example 2, are interpenetrated by hydrophilic materials such as polyethylene glycol, poly(vinyl alcohol), poly(2-hydroxyethyl methacrylate), alginate, chitosan through the swelling of tofu thermoplastics of Examples 1 and 2 in water or alcohol additive solutions. Following interpenetration a further dehydration step is carried out to allow physical interactions between the two components of the composite material. Dehydration is performed at different temperatures according to the stability of the additive. The obtained specimens can be functionalised as reported in Example 3.
   (iv) Physical cross-linking can be obtained introducing hydrophobic domains in the paste either as described in point (i) or as described in Example 3. Alternatively, salt bridges can be generated introducing molecules of opposite charges. In the first case, blends of the fresh tofu cheese with synthetic and natural polymers able to form hydrophobic interactions or hydrogen bonding are carried out as reported in points (i) and (iii). Grafting of monaldehydes bearing hydrophobic, hydrophilic or charged groups can also be performed as reported in Example 3.
   (v) Blending of the tofu paste with hydroxylapatite and calcium phosphates at different ratios prior to thermosetting can be performed to increase the osteointegrative properties of the tofu-based thermoplastics and thermoset products.

### EXAMPLE 5: Tofu thermoplastic/thermoset biomaterials for wound dressing and other applications.

### METHOD

Tofu-based sponges with different porosity are prepared as reported in Examples 1, 2 and 4. Rectangular (1.0 cm x 0.5 cm) compact samples were incubated in phosphate buffer at 37 °C and their weight was determined at different times after removal of the excess of buffer from the surface by capillarity.

After complete swelling, the specimens were tested for their mechanical properties by Instron according to ISO standards.

### RESULTS

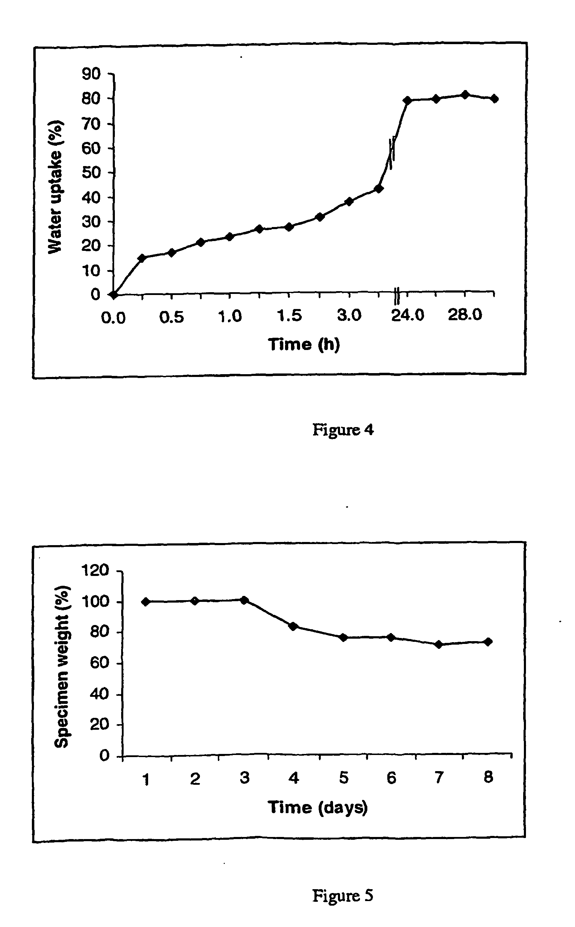

Films with a low porosity and a thickness of 1.0 mm have a relatively rapid relaxation time and allow to adsorb wound exudates for long period of times (approximately 24 h) (Figure 4).

Water uptake can be modulated by changing the physical-chemical properties of the sponges as reported in Examples 1, 2 and 4.

The mechanical properties of the wet thermoplastic films are summarised in Table 1. The good elastic properties of this material at wet conditions are emphasised.

**Table 1. Mechanical properties of wet tofu thermoplastic films.**

| | Parameter mean (range) | | |
|---|---|---|---|
| Sample number | Stress at break (Mpa) | Elongation at break (mm/mm) | Modulus (Mpa) |
| 3 | 0.921 (0.842-1.068) | 0.905 (0.796-0.992) | 3.643 (3.140-3.957) |

Example 6: Tofu thermoplastic/thermoset biomaterials as biodegradable barriers to prevent post-surgical tissue adherence and other applications.

### METHOD

Tofu-based biodegradable films functioning as a temporary barrier to prevent post-surgical tissue adherence and other applications in which degradation is required can be obtained by the preparation methods described in Examples 1, 2 and 3. The membranes, as such or modified with specific functional groups as reported in Examples 3 and 4, can also be applied in dental practise, aesthetic surgery and other kinds of applications where the growth of soft and bone tissues has to be modulated.

Rectangular (1.0 x 0.5 cm) specimens were incubated in phosphate buffer for 9 days at 37 °C. The specimens were weighed at different time after removal of the excess of buffer from the surface by capillarity.
After 7 days incubation in buffer, specimens were washed in de-ionised water, freeze-dried overnight, sputter-coated with gold and analysed by Scanning Electron Microscopy at x200, 5 kV.

### RESULTS

The compact films produced following the method described in Example 1 showed a slow and spontaneous degradation in an aqueous environment (Figure 5). The degradation induced by spontaneous hydrolysis is very slow, accounting for 30% of the specimen weight after 9 days.

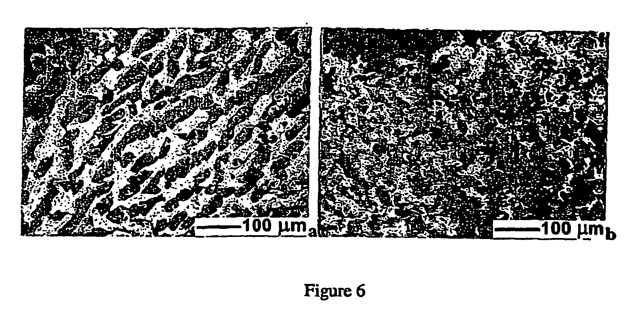

The degradation of the film after 7 days led to the formation of a highly porous gel-like film (Figure 6 a) easily removed from the bulk of the material which showed a different degree of porosity (Figure 6 b). Both the degrees of porosity obtained were in the range required for tissue in-growth (100-500 µm).

The intrinsically slow biodegradation of the tofu thermoplastics of different morphologies also suggests its use as a carrier for slow delivery of antibiotics and drugs.

### EXAMPLE 7: Tofu thermoplastics/thermoset products as osteointegrative biomaterials.

### METHOD

The presence of relatively high amounts of calcium used as a coagulant agent in the preparation of tofu cheese (Example 1), the modulated porosity of the hydrogels (Example 2) and their functionalisation with bioactive peptides (Example 3) suggest the use of these materials as a substratum for osteointegration.

Compact tofu films were incubated in a buffer solution with a salt composition simulating the bone exudates for 2 days, 37 °C. Control disks were incubated in PBS for the same length of time. The specimens were washed in de-ionised water and frozen. The sample aqueous phase was sublimed in a Cryostat preparation chamber, and the dried material sputter-coated with palladium and analysed in frozen state by Cryo-Scanning Electron Microscopy. The morphology was analysed at x3,000, 5 kV; elemental analysis was carried out at 15 kV.

### RESULTS

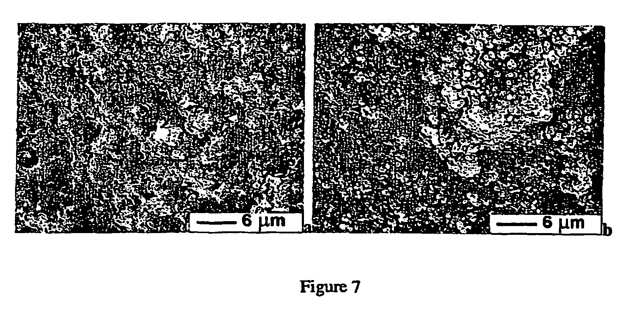

The formation of a consistent mineral phase on the surface of a non-modified tofu thermoplastic film was visible already after 2 days incubation in simulated body fluid buffer (Figure 7 b). In the same experimental conditions a potent osteointegrative material such as Polyactive® (CAM, The Netherlands) did not show any detectable mineralization (data not shown).

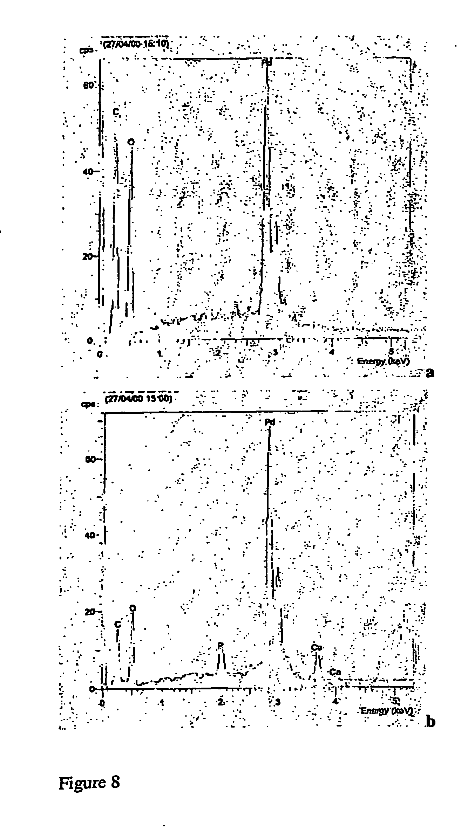

Elemental analysis allowed characterisation of the mineral phase as calcium phosphate in which the Ca/P ratio was 1.05 (Figure 8 b). Irrespective of the film preparation method no detectable phosphorus and calcium peaks were found in the control (Figure 8 a). The peaks of the carbon and oxygen atoms, typical of the organic soybean polymers, decreased when the material were incubated in simulating body fluid clearly indicating that the formed mineral phase reached a significant thickness (Figures 8 a and b).

The control specimen (Figure 7 a) exposed to a PBS medium showed a change of the surface morphology which became relatively smoother than the dried material (Figure 1 b) similarly to the pressure-expanded materials (Figures 2 d-f).

### EXAMPLE 8: Anti-inflammatory properties of tofu thermoplastics.

### METHOD

Tofu films, previously wetted in physiological solution and then conditioned with human plasma for 30 min at 37 °C, static conditions, were analysed by Enzyme-Linked Immuno Assay (ELISA) for the adsorption of proteins with a key role in the wound healing process. The results were compared with those obtained from two synthetic materials, polystyrene (PST) and poly(2-hydroxyethyl methacrylate) (PHEMA), with completely different physico-chemical surface properties.
The effect of tofu-based thermoplastics and their degradation products on the inflammatory cells was also evaluated. Mononuclear cells (10⁵/ml), separated from human peripheral blood by Boyum's method⁹, were added to 3 ml of phosphate buffer in which a swollen rectangular (1.0 cm x 0.5 cm) specimen was previously placed. The effect of the degradation products was also evaluated by adding to the buffer 100 µl of the incubation medium in which film degradation was carried out for 3 days. In both cases Luminol 1.0 mM was previously added to the incubation medium. Chemiluminescence, induced by the production of free radicals by the cells, was continuously detected for 1 h.

In an alternative method, mononuclear cells (10⁵) were incubated in the presence of a 1.7 cm diameter material disk, previously conditioned with human plasma, for 20 h, 37 °C, 95% air, 5% CO₂. Supernatants were withdrawn, centrifuged at 1,000 g to avoid the contamination of not bound cell, and tested by an ELISA kit for Interleukin-1β.

In a similar experiment, the cells adhering to the plasma-conditioned material were fixed to the surface by 2.5% (w/v) glutaraldeyde solution in PBS. After fixing, the material was gradually dehydrated by incubation in ethanol/PBS solutions of increasing alcohol concentration (25%, 50%, 75% by volume). Two final steps in absolute ethanol were finally performed. All the steps lasted 15 min at 4 °C. The specimens were freeze-dried overnight, sputter-coated with gold and analysed by Scanning Electron Microscopy, at different magnifications, 5 kV.

### RESULTS

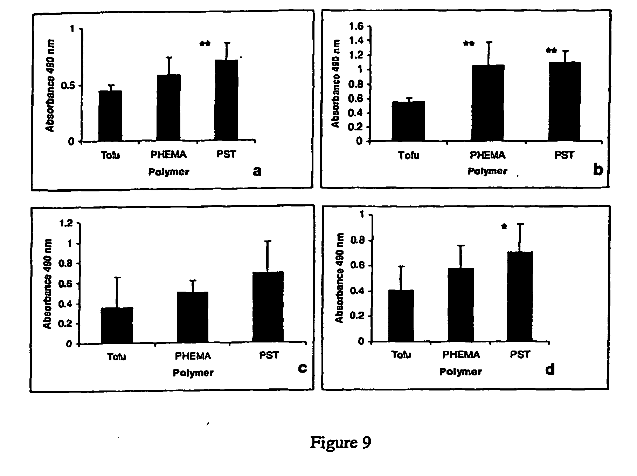

ELISA showed that tofu-based thermoplastic surface bound levels of C3 fragment of complement system at levels significantly lower than PST (Figure 9 a). Fibronectin also adsorbed at levels which were significantly lower than PST (Figure 9 d), whereas no significant difference was found when the levels of immunoglobulins (IgG) were evaluated (Figure 9 c). A significant decrease of Fibrinogen was found with respect to the two synthetic materials (Figure 9 b).

The activation of mononuclear cells isolated from peripheral human blood was also affected. Tofu films, whose surface was pre-conditioned with human plasma, induced a very low production of interleukin-1β compared with PST and PHEMA (Table 2).

**Table 2 Material-induced synthesis of Interleukin-1β by mononuclear cells. Experiments were carried out in triplicate.**

| Sample (n=3) | Il-1β concentration mean (pg/ml) | Value range |
|---|---|---|
| Tofu | 56.2 | 23.2-89.2 |
| PHEMA | 256.9 | 220.7-276.1 |
| PST | 331.0 | 179-545 |

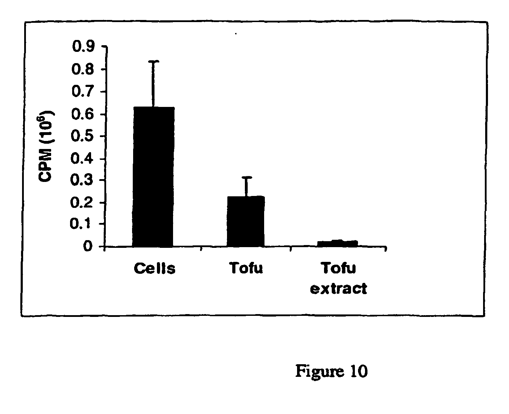

The evaluation of the free radical production by the mononuclear cells through a chemiluminescence method showed that tofu thermoplastics as well as their degradation products significantly suppressed the spontaneous activation of mononuclear cells (Figure 10). The incubation of the monocytes with the tofu extracts derived from degradation experiments in physiological solution showed an even stronger inhibitory effect on the free radical production.

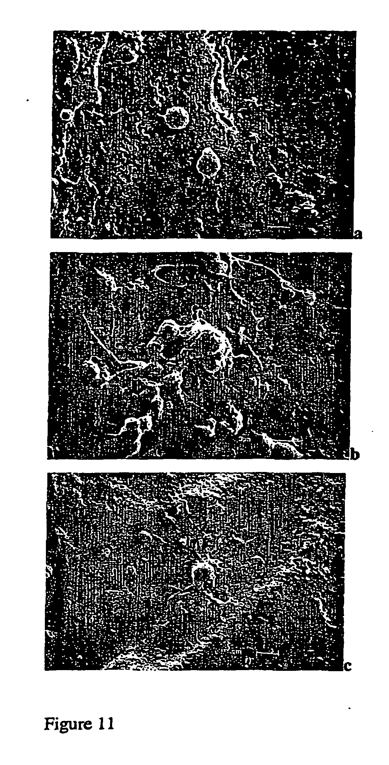

Finally, the adhesion of the monocytes/macrophages on tofu-derived thermoplastic films was also limited to round-shaped cells with a smooth surface indicating their quiescent state (Figures 11 a-c). The adhesion of the cells was poor with only tiny pseudopodia establishing contacts with the surface of the material (Figures 11 a and 11 b). Only rarely a good cell spreading was observed (Figure 11 c), but the macrophages still showed a smooth surface typical of non-active cells.

### EXAMPLE 9: Tofu thermoplastics as scaffold for tissue engineering

### METHOD

Tofu thermoplastic disks (1-cm diameter in dry state) were disinfected with 90% ethanol for 15 min. The specimens were equilibrated for 1 h in cell culture growth medium enriched with 10% (v/v) fetal bovine serum and 3T3 murine fibroblasts (2.5 x 10⁵) were seeded onto each disk. Cell adhesion/proliferation was stopped at 6 h, 24 h and 48 h incubation time at 37 °C, 95% air, 5% CO₂. For each incubation time controls in tissue culture plates (TCP) were also performed. Adhering cells were fixed and dehydrated as reported in the previous example and samples were sputter-coated by gold and analysed by Scanning Electron Microscopy at different magnification at 5 keV.

### RESULTS

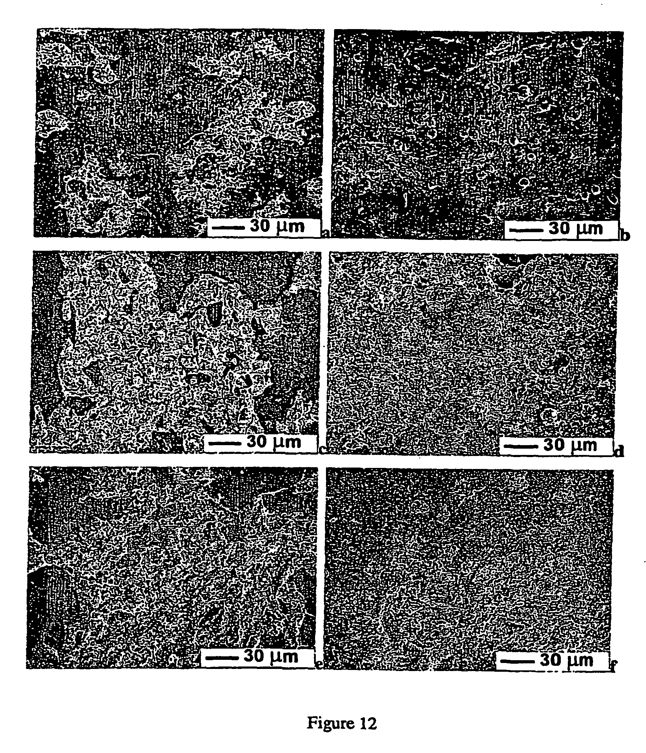

Figures 12 a and b show the degree of adhesion and proliferation of the fibroblasts on the TCP (a) and soybean-based thermoplastic (b) after 6 h of incubation. A poor degree of cell spreading was detected on the tofu-based materials; the fibroblasts appeared still round shaped and only tiny lamellipodia established contacts with the surface. Conversely, the spreading of the cells on the TCP was already completed after the same length of time.

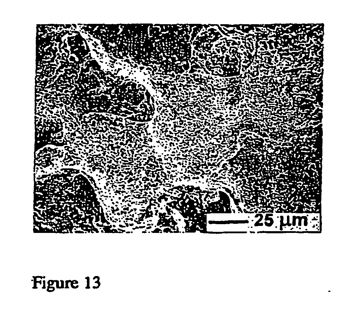

However, at 24 h and 48 h, the fibroblasts reached a degree of spreading on the tofu thermoplastics (Figures 12 d and f) comparable to that of the control (c and e). The cells formed confluent layers with a new extracellular matrix covering the surface of the biomaterial. Few areas of isolated cells allowed to highlight the high degree of spreading achieved by the cells on tofu-based biomaterials after 24 h (Figure 13).

### REFERENCES

1. Santin, M., Huang, S. J., lannace, S., Ambrosio, L., Nicolais, L., Peluso, G. 1996 Synthesis and characterization of a new interpenetrated poly(2-hydroxyethylmethacrylate)-gelatin composite polymer. Biomaterials 17: 1459-1467.
2. Kim, H. J., Lee, H. C., Oh, J. S., Shin, B. A., Oh, C. S., Park, R. D., Yang, K. S., Cho, C. S. 1999 Polyelectrolyte complex composed of chitosan and sodium alginate for wound dressing application. J. Biomat. Sci.-Polymer Ed. 10: 543-556.
3. Jalil, R., Nixon, J. R. 1990 Biodegradable poly(lactic acid) and poly(lactide-co-glycolide) microcapsule: problems associated with preparative techniques and release properties J. Microencapsulation 7, 297-325.
4. Patent No. US5523293, 1996.
5. Vaz, C. M., Foseen, M., Cunha, A. M., Reis, R. L., Casein and soybean thermoplastic proteins as alternative biodegradable polymers for biomedical applications. Sixth World Biomaterials Congress, Kamuela, USA, 15-20 May, 2000, Abstract 429.
6. Patent No. US006042851A, 1998.
7. Patent No. US5674548, 1997.
8. Saio, K, Watanabe, T. 1973 Food use of soybean 7S and 11S proteins. Extraction and functional properties of their fractions. Journal of Food Science 38, 1139-1144.
9. Boyum A. 1968 Isolation of mononuclear cells and granulocytes from human blood. Scand J Clin Lab Invest; 21: 77-89.

## Claims

1. Use as a biomaterial of a compact or porous thermoset product made of soybean de-fatted tofu.

2. Use of a compact or porous thermoset product made of soybean de-fatted tofu for the manufacture of a biomaterial.

3. Use as claimed in claim 1 or claim 2, wherein the biomaterial is in the form of or for use in a wound dressing.

4. Use as claimed in claim 1 or claim 2, wherein the biomaterial is in the form of or for use in a temporary barrier to prevent post-surgical tissue adherence.

5. Use as claimed in claim 1 or claim 2, wherein the biomaterial is in the form of or for use in a filler or coating material with osteointegrative potential.

6. Use as claimed in claim 1 or claim 2, wherein the biomaterial or a degradation product thereof is in the form of or for use in an anti-inflammatory agent.

## Patentansprüche

1. Verwendung eines kompakten oder porösen wärmehärtbaren Produkts, hergestellt aus entfettetem Sojatofu, als Biomaterial.

2. Verwendung eines kompakten oder porösen wärmehärtbaren Produkts, hergestellt aus entfettetem Sajatofu für die Herstellung eines Biomaterials.

3. Verwendung nach Anspruch 1 oder 2, wobei das Biomaterial in der Form von oder zur Verwendung in einem Wundverband ist.

4. Verwendung nach Anspruch 1 oder 2, wobei das Biomaterial in der Form oder zur Verwendung in einer zeitweiligen Sperre zur Verhinderung von nachchirurgischem Gewebsanhaften ist.

5. Verwendung nach Anspruch 1 oder 2, wobei das Biomaterial in der Form von oder zur Verwendung in einem Füllstoff oder Beschichtungsmaterial mit osteointegrativem Potenzial ist.

6. Verwendung nach Anspruch 1 oder 2, wobei das Biomaterial oder ein Abbauprodukt davon in der Form von oder zur Verwendung in einem entzündungshemmenden Mittel ist.

## Revendications

1. Utilisation en tant que biomatériau d'un produit thermodurci compact ou poreux constitué de tofu dégraissé de soja.

2. Utilisation d'un produit thermodurci compact ou poreux constitué de tofu dégraissé de soja pour la fabrication d'un biomatériau.

3. Utilisation selon la revendication 1 ou la revendication 2, dans laquelle le biomatériau est sous la forme de ou pour une utilisation dans un bandage.

4. Utilisation selon la revendication 1 ou la revendication 2, dans laquelle le biomatériau est sous la forme de ou pour une utilisation dans une barrière temporaire destinée à prévenir l'adhérence tissulaire post-chirurgicale.

5. Utilisation selon la revendication 1 ou la revendication 2, dans laquelle le biomatériau est sous la forme de ou pour une utilisation dans un matériau de remplissage ou de revêtement ayant une capacité ostéo-intégrative.

6. Utilisation selon la revendication 1 ou la revendication 2, dans laquelle le biomatériau ou un produit de dégradation de celui-ci est sous la forme de ou pour une utilisation dans un agent anti-inflammatoire,
